# EUROPÄISCHE PATENTSCHRIFT

(11) **EP 0 715 864 B1**
(45) Veröffentlichungstag und Bekanntmachung des Hinweises auf die Patenterteilung: **18.09.2002**
(21) Anmeldenummer: 95119205.3
(22) Anmeldetag: 06.12.1995
(51) Int. Cl.: A61M 39/20

(54) **Vorrichtung zum Verschliessen einer medizinischen Leitung, z.B. der eines Katheters**
Closure device of a medical conduit, for instance of a catheter
Dispositif de fermeture d'un conduit médical, par exemple d'un cathéter

(30) Priorität: 09.12.1994 DE 9419630 U
(43) Veröffentlichungstag der Anmeldung: 12.06.1996
(73) Patentinhaber: Fresenius Medical Care Deutschland GmbH, 61350 Bad Homburg v.d.H. (DE)
(72) Erfinder: Heilmann, Klaus, DE-66606 St.Wendel (DE); Jessen, Claus Dr, DE-66620 Otzenhausen (DE)
(74) Vertreter: Vièl, Georg, Dipl.-Ing.

(56) Entgegenhaltungen:
- WO-A-83/03975
- BE-A- 350 384
- GB-A- 527 140
- US-A- 4 394 923
- US-A- 4 597 758

## Beschreibung

Die Erfindung betrifft eine Kombination einer Schutzkappe, welche Verbindungsmittel zum Verbinden der Schutzkappe mit einem Gegenstück aufweist, mit einem Verschlußelement.

Schutzkappen z.B. zum Abschließen von Infusionsleitungen sind bekannt und werden beispielsweise bei der Peritonealdialyse zum Verschließen eines Konnektors oder eines Kupplungsstücks am Bauchkatheter eines Patienten verwendet. Dort wird beispielsweise nach Durchführung eines Beutelwechsels der Konnektor mit einer Schutzkappe gesichert, die am Konnektor festgeschraubt wird.

Soll nun ein weiterer Beutelwechsel durchgeführt werden, muß zunächst die Schutzkappe abgeschraubt und dann das Verschlußstück entfernt werden. Gerade das Entfernen des Verschlußstücks ist jedoch problematisch, da dabei einerseits leicht Keime auf den Konnektor und damit auch in den Bauchraum des Patienten übertragen werden können, was zu einer Peritonitis führen kann und andererseits das Verschlußelement relativ fest in dem Konnektor sitzt, so daß es schwierig zu entfernen ist.

Aus der WO 83/03975 ist eine Schutzkappe insbesondere für die Peritonealdialyse bekannt, bei der durch einen mit Desinfektionsmittel getränkten Schwamm das Ende des Konnektors keimfrei gehalten wird. Mit dieser Schutzkappe kann jedoch nicht ein Verschlußelement entfernt werden.

Aufgabe der Erfindung ist es somit, eine Vorrichtung zum Abschließen einer Leitung gemäß dem Oberbegriff zu schaffen, mit der in einem Schritt sowohl ein Verschlußelement als auch die Schutzkappe selbst von der Leitung entfernt werden können.

Diese Aufgabe wird erfindungsgemäß durch die im Anspruch 1 beschriebene Kombination gelöst.

Durch die ersten Verbindungsmittel kann die Schutzkappe unlösbar mit einem Verschlußelement verbunden werden; beim Entfernen der Schutzkappe wird somit das Verschlußelement mit entfernt, ohne daß es berührt werden muß. Die weiteren Verbindungsmittel sind zum lösbaren Verbinden der Schutzkappe mit der Leitung vorgesehen. Dadurch wird die Schutzkappe gehalten und ein versehentliches Entfernen des Verschlußelements vermieden.Dem Patienten wird also ein zusätzlicher Handlingsschritt, nämlich das Entfernen des Verschlußelements, erspart, und er braucht das Verschlußelement nicht zu berühren, so daß eine Gefahr einer Verkeimung der Leitung entfällt.

Eine Weiterbildung der Erfindung besteht darin, daß an der Schutzkappe Mittel zum Freisetzen eines Antiseptikums durch den Druck des Verschlußelementes beim Aufbringen der Schutzkappe vorgesehen sind.

Erfindungsgemäß ist vorgesehen, daß das erste Verbindungsmittel aus mindestens einem Einrastelement, einem nur in eine Richtung wirkenden Schraubverschluß oder einem Sägezahndrehelement (Ratsche) besteht.

Diese Verbindungsmittel sind - neben anderen - dazu geeignet, eine unlösbare Verbindung zwischen zwei Teilen herzustellen.

Bei der Verwendung eines Einrastelements als Verbindungsmittel kann vorgesehen sein, daß dieses aus mindestens einem Vorsprung zum Eingriff in eine Nut oder aus mindestens einer Nut zum Zusammenwirken mit einem Vorsprung besteht.

Das weitere Verbindungsmittel kann beispielsweise aus einem Schraubverschluß, einer Steckverbindung oder einem Bajonettverschluß bestehen.

Bei diesen Ausführungen wird der Kraftaufwand zum Entfernen des Verschlußelements z.B. durch die Gewindeübersetzung, gering gehalten. Beim Aufschrauben der Schutzkappe auf das Verschlußelement rastet ein Einrastelement in den Verschlußstopfen ein und wird beim Entfernen der Schutzkappe von der Leitung mitentfernt.

Als Mittel zum Freisetzen eines Antiseptikums kann ein mit Antiseptikum getränktes Adsorptionsmaterial, beispielsweise ein Schaumpolymer oder ein Schwamm, vorgesehen werden.

Im Rahmen der im Anspruch 1 beschriebenen Erfindung liegt auch ein Verschlußelement zum Verschließen einer Leitung, das mindestens ein Verbindungsmittel zum unlösbaren Verbinden des Verschlußelements mit einer erfindungsgemäßen Schutzkappe aufweist.

Erfindungsgemäß, siehe Anspruch 13, ist weiterhin ein Verfahren zum Entfernen eines Verschlußelements aus einer Leitung, bestehend aus folgenden Verfahrensschritten:
- Aufbringen der Schutzkappe über das Verschlußelement, wobei eine lösbare Verbindung zwischen der Schutzkappe und der Leitung und eine unlösbare Verbindung zwischen der Schutzkappe und dem Verschlußelement geschaffen werden,
- Entfernen der Schutzkappe von der Leitung, wobei das Verschlußelement mit der Schutzkappe verbunden bleibt.

Weiterhin ist erfindungsgemäß die Verwendung einer erfindungsgemäßen Kombination zum Abschließen eines Peritonealkatheters gemäß Anspruch 14.

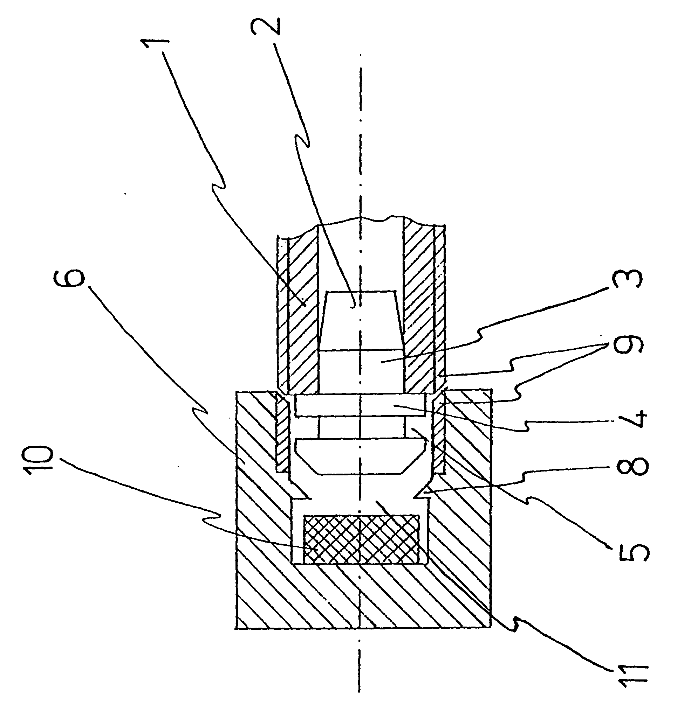

Im folgenden wird die Erfindung anhand einer Zeichnung beschrieben. Die Zeichnung zeigt eine geschnittene Darstellung eines Beispiels einer erfindungsgemäßen Vorrichtung.

In einer Leitung 1, die beispielsweise ein Kupplungsstück oder ein Konnektor sein kann, ist ein Verschlußelement 2 derart eingebracht, daß der erste Bereich 3 des Verschlußelements 2 die Leitung 1 flüssigkeitsdicht verschließt. Der zweite Bereich 4 des Verschlußelements 2, der das "Kopfstück" des Verschlußelements 2 darstellt, weist Mittel 5 zum Verbinden des Verschlußelements 2 mit einer Schutzkappe 6 auf.

Diese Mittel 5 zum Verbinden des Verschlußelements 2 und der Schutzkappe 6 können beispielsweise, wie hier dargestellt, eine Nut 7 im zweiten Bereich 4 des Verschlußelements 2 und ein der Nut 7 entsprechender Vorsprung 8 an der Innenseite der Schutzkappe 6 sein. Beim Aufbringen der Schutzkappe 6 über das Verschlußelement 2, was über geeignete Mittel 9 zum Verbinden der Schutzkappe 6 mit der Leitung 1, beispielsweise über an der Schutzkappe 6 und dem Verschlußelement 2 angeordnete Gewinde, erfolgt, in dem dargestellten Beispiel durch ein Aufschrauben der Schutzkappe 6 auf die Leitung 1, greift so der Vorsprung 8 in die Nut 7 und Verschlußelement 2 und Schutzkappe 6 sind verbunden. Selbstverständlich können auch beliebige andere Mittel 5 zum Verbinden des Verschlußelements 2 und der Schutzkappe 6 gewählt werden, z.B. auch eine Nut in der Schutzkappe 6 und ein Vorsprung an dem Verschlußelement 2, ein nur in eine Richtung wirkender Schraubverschluß oder auch ein Sägezahndrehelement (auch Ratsche genannt). Es kann dabei, wie dargestellt, sinnvoll sein, daß das Verschlußelement 2 am oberen Ende seines zweiten Bereichs 4 und/oder der Vorsprung 8 an der dem Verschlußelement 2 zugewandten Seite abgeschrägt sind, um das Einrasten zu erleichtern.

Weiterhin ist es vorgesehen, daß die Schutzkappe 6 in ihrem Innenbereich 11 ein mit Antiseptikum getränktes Absorptionsmaterial 10, z.B. einen Schwamm, aufweist, das sich beim Aufbringen der Schutzkappe 6 über das Verschlußelement 2 durch den Druck des oberen Bereichs 4 des Verschlußelements 2 entleert und den Innenbereich 11, der durch Luftkeime verunreinigt sein kann, desinfiziert. Statt des Schwammes kann z.B. auch ein mit Antiseptikum gefülltes Bläschen vorliegen, das beim Verbinden der Schutzkappe 6 mit der Leitung 1 platzt. Das Absorptionsmaterial 10 kann beispielsweise mit Jod/Jodid oder anderen geeigneten Desinfektionsmitteln, wie z.B. den von der Anmelderin vertriebenen "Citrosteril" auf Zitronensäure-Basis oder "Lavasept" auf Biguanid-Basis gefüllt sein. Weitere verwendbare Desinfektionsmittel sind Peroxidverbindungen, Ozon oder Hypochlorit, die auch in situ herstellbar sein können. Vorteilhafterweise wird das Antiseptikum erst beim Aufschrauben der Schutzkappe auf die Leitung in den nach dem Verbindungsvorgang geschlossenen Innenbereich 11 freigesetzt.

Am Ende des Beutelwechselvorgangs einer Peritonealdialyse wird somit durch eine letzte Drehung des Drehstücks das Verschlußelement 2, ohne daß dieses berührt werden muß, in die Leitung 1 eingebracht und diese verschlossen. Anschließend wird die Schutzkappe 6 über weitere Verbindungsmittel 9 (hier: Gewinde), mit der Leitung 1 lösbar verbunden, wobei das erste Verbindungsmittel der Schutzkappe 6 mit dem Verbindungsmittel 5 des Verschlußelements 2 zusammenwirkt und das im Schwamm 10 enthaltene Antiseptikum sich in den verschlossenen Innenbereich 11 entleert. Der Innenbereich 11 liegt damit zwischen zwei Beutelwechseln keimfrei vor. Beim nächsten Beutelwechsel wird die Schutzkappe 6 abgeschraubt und nimmt das Verschlußelement 2 mit, was aufgrund der Gewindeübersetzung nur einen geringen Kraftaufwand erfordert. Ein Berühren der Leitung 1 oder des Verschlußelements 2 entfällt mit diesem System somit völlig, womit ein wichtiger Beitrag zur Sicherheit der Peritonealdialyse geleistet wurde.

## Patentansprüche

1. Kombination einer Schutzkappe (6) zum Abschließen einer Infusionsleitung oder eines Katheters (1) mit einem Gegenstück (2), wobei die Schutzkappe (6) mindestens ein erstes Verbindungsmittel (8) zum Verbinden der Schutzkappe (6) mit dem Gegenstück (2) und mindestens ein weiteres Verbindungsmittel (9) aufweist, und das Gegenstück (2) mindestens ein Verbindungsmittel (5) zum Verbinden mit der Schutzkappe aufweist, **dadurch gekennzeichnet,**
**daß** das Gegenstück (2) ein die Infusionsleitung oder den Katheter (1) flüssigkeitdicht abschließendes Verschlußelement (2) ist, das erste Verbindungsmittel (8) unlösbar mit dem Verbindungsmittel (5) des Verschlußelementes (2) beim Aufbringen der Schutzkappe über das Verschlußelement verbindbar ist und das weitere Verbindungsmittel (9) mit der Infusionsleitung oder dem Katheter (1) lösbar verbindbar ist.

2. Kombination gemäß Anspruch 1, **dadurch gekennzeichnet, daß** an der Schutzkappe (6) Mittel (10) zum Freisetzen eines Antiseptikums durch den Druck des Verschlußelementes beim Aufbringen der Schutzkappe (6) vorgesehen sind.

3. Kombination gemäß Anspruch 1, **dadurch gekennzeichnet, daß** das erste Verbindungsmittel (8) der Schutzkappe (6) aus mindestens einem Einrastelement besteht.

4. Kombination gemäß Anspruch 1, **dadurch gekennzeichnet, daß** das erste Verbindungsmittel (8) der Schutzkappe (6) aus einem nur in eine Richtung wirkenden Schraubverschluß besteht.

5. Kombination gemäß Anspruch 1, **dadurch gekennzeichnet, daß** das erste Verbindungsmittel (8) der Schutzkappe (6) aus einem Sägezahndrehelement besteht.

6. Kombination gemäß Anspruch 3, **dadurch gekennzeichnet, daß** das Einrastelement aus mindestens einem Vorsprung zum Eingriff in eine Nut besteht.

7. Kombination gemäß Anspruch 3, **dadurch gekennzeichnet, daß** das Einrastelement aus mindestens einer Nut zum Zusammenwirken mit einem Vorsprung besteht.

8. Kombination gemäß Anspruch 1, **dadurch gekennzeichnet, daß** das weitere Verbindungsmittel (9) der Schutzkappe (6) aus einem Schraubverschluß, einer Steckverbindung oder einem Bajonettverschluß besteht.

9. Kombination gemäß Anspruch 2, **dadurch gekennzeichnet, daß** die Mittel (10) zum Freisetzen eines Antiseptikums aus einem mit Antiseptikum getränkten Adsorptionsmaterial bestehen.

10. Kombination gemäß Anspruch 9, **dadurch gekennzeichnet, daß** das Adsorptionsmaterial ein Schaumpolymer oder ein Schwamm ist.

11. Kombination gemäß Anspruch 1, **dadurch gekennzeichnet, daß** das Verbindungsmittel (5) des Verschlußelements (2) aus mindestens einem Einrastelement besteht.

12. Kombination gemäß Anspruch 11, **dadurch gekennzeichnet, daß** das Einrastelement des Verschlußelements (2) ein Gegenstück zu einem Einrastelement der Schutzkappe (6) ist.

13. Verfahren zum Entfernen eines Verschlußelements (2) aus einer Infusionsleitung oder einem Katheters (1) mittels einer Schutzkappe (6), bestehend aus folgenden Verfahrensschritten:
• Aufbringen der Schutzkappe (6) über das Verschlußelement (2), wobei eine lösbare Verbindung zwischen der Schutzkappe (6) und der Infusionsleitung oder dem Katheter (1) und eine unlösbare Verbindung zwischen der Schutzkappe (6) und dem Verschlußelement (2) geschaffen werden und Antiseptikum im Innenbereich der Schutzkappe (6) freigesetzt wird,
• Entfernen der Schutzkappe (6) von der Infusionsleitung oder dem Katheter (1), wobei das Verschlußelement (2) mit der Schutzkappe (6) verbunden bleibt.

14. Verwendung einer Kombination gemäß den Ansprüchen 1 bis 12 zum Abschließen eines Peritonealkatheters.

## Claims

1. Combination of a protective cap (6) for sealing off an infusion conduit or a catheter (1) and a counterpart (2), the protective cap (6) having at least one connecting means (8) for connection of the protective cap (6) with a counterpart (2) and at least one additional connecting means (9), and the counterpart (2) having at least one connecting means (5) for connecting it with the protective cap,
**characterised in that**
the counterpart (2) is a closure element (2) for liquid-tight closure of an infusion conduit or catheter (1), the first connecting means (8) connects permanently with the connecting means (5) of the closure element (2) when the protective cap is fitted over the closure element, and the additional connecting means (9) is releasably connectable with the infusion conduit or catheter (1).

2. The combination of claim 1, **characterised in that** the protective cap (6) has attached to it a means (10) which releases an antiseptic when pressure is exerted by the closure element during fitting of the protective cap (6) over the closure element.

3. The combination of claim 1, **characterised in that** the first connecting means (8) of the protective cap (6) consists of at least one snap-in member.

4. The combination of claim 1, **characterised in that** the first connecting means (8) of the protective cap (6) consists of a unidirectionally operative threaded element.

5. The combination of claim 1, **characterised in that** the first connecting means (8) of the protective cap (6) consists of a ratchet wheel.

6. The combination of claim 3, **characterised in that** the snap-in member consists of at least one projection for engagement with a groove.

7. The combination of claim 3, **characterised in that** the snap-in element consists of at least one groove for coaction with a projection.

8. The combination of claim 1, **characterised in that** the additional connecting means (9) of the protective cap (6) consists of a threaded element, a plug connection or a bayonet lock interface.

9. The combination of claim 2, **characterised in that** the means (10) for releasing an antiseptic consists of absorptive material saturated with antiseptic.

10. The combination of claim 9, **characterised in that** the absorptive material is a foamed polymer or a sponge.

11. The combination of claim 1, **characterised in that** the connecting means (5) of the closure element (2) consists of at least one snap-in member.

12. The combination of claim 11, **characterised in that** the snap-in member of the closure element (2) is a counterpart to a snap-in member of the protective cap (6).

13. A method of disconnecting a closure element (2) from an infusion conduit or catheter (1) by means of a protective cap (6), said method comprising the following steps:
• Fitting the protective cap (6) over the closure element (2), thereby creating a releasable connection between the protective cap (6) and the infusion conduit or catheter as well as a permanent connection between the protective cap (6) and the closure element (2), and causing antiseptic to be released in the interior of the protective cap (6).
• Disconnecting the protective cap (6) from the infusion conduit or catheter (1); the closure element (2) remains connected with the protective cap (6).

14. Use of a combination according to claims 1 to 12 for sealing off a peritoneal catheter.

## Revendications

1. Combinaison d'un capuchon de protection (6) pour fermer un conduit de perfusion ou d'un cathéter (1) avec un élément complémentaire (2), le capuchon de protection (6) étant muni d'au moins un premier moyen de liaison (8) pour relier ensemble le capuchon de protection (6) et l'élément complémentaire (2) et au moins un second moyen de liaison (9), et l'élément complémentaire (2) étant muni d'au moins un moyen de liaison (5) pour le relier au capuchon de protection, **caractérisée en ce que** l'élément complémentaire (2) est un élément de fermeture (2) fermant de façon étanche la conduite de perfusion ou le cathéter (1), le premier moyen de liaison (8) peut être relié de façon définitive avec le moyen de liaison (5) de l'élément de fermeture (2) lorsque le capuchon de protection (6) est mis sur l'élément de fermeture et le moyen de liaison supplémentaire (9) peut être relié de façon non définitive au conduit de perfusion ou au cathéter (1).

2. Combinaison selon la revendication 1, **caractérisée en ce qu'**il est prévu sur le capuchon de protection (6) des moyens (10) pour libérer un antiseptique sous l'effet de la pression de l'élément de fermeture lors de la mise en place du capuchon de protection.

3. Combinaison selon la revendication 1, **caractérisée en ce que** le premier moyen de liaison (8) du capuchon de protection (6) est composé d'au moins un élément d'encliquetage.

4. Combinaison selon la revendication 1, **caractérisée en ce que** le premier moyen de liaison (8) du capuchon de protection (6) est composé d'un obturateur à vis ne pouvant agir que dans un sens.

5. Combinaison selon la revendication 1, **caractérisée en ce que** le premier moyen de liaison (8) du capuchon de protection (6) se compose d'un élément rotatif en dent de scie.

6. Combinaison selon la revendication 3, **caractérisée en ce que** l'élément d'encliquetage se compose d'au moins d'une saillie destinée à pénétrer dans une gorge.

7. Combinaison selon la revendication 3, **caractérisée en ce que** l'élément d'encliquetage se compose d'au moins une gorge destinée à coopérer avec une saillie.

8. Combinaison selon la revendication 1, **caractérisée en ce que** le moyen de liaison supplémentaire (9) du capuchon de protection (6) se compose d'une fermeture filetée, d'une connexion par fiche ou d'une fermeture à baïonnette.

9. Combinaison selon la revendication 2, **caractérisée en ce que** le moyen (10) pour libérer un antiseptique se compose d'un matériau adsorbant imprégné d'antiseptique.

10. Combinaison selon la revendication 9, **caractérisée en ce que** le matériau adsorbant est un polymère expansé ou une éponge.

11. Combinaison selon la revendication 1, **caractérisée en ce que** le moyen de liaison (5) de l'élément de fermeture (2) se compose d'au moins un élément d'encliquetage.

12. Combinaison selon la revendication 11, **caractérisée en ce que** l'élément d'encliquetage de l'élément de fermeture (2) est un élément complémentaire à un élément d'encliquetage du capuchon de protection (6).

13. Procédé pour enlever un élément de fermeture (2) d'un conduit de perfusion ou d'un cathéter (1) au moyen d'un capuchon de protection (6) comportant les étapes suivantes :
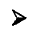 mise en place du capuchon de protection (6) sur l'élément de fermeture (2), une liaison réversible entre le capuchon de protection (6) et le conduit de perfusion ou le cathéter (1) et une liaison irréversible entre le capuchon de protection (6) et l'élément de fermeture (2) étant réalisées et un antiseptique étant libéré dans la zone intérieure du capuchon de protection (6),
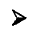 enlèvement du capuchon de protection (6) du conduit de perfusion ou du cathéter (1), l'élément de fermeture (2) restant relié au capuchon de protection (6).

14. Utilisation d'une combinaison selon l'une des revendications 1 à 12 pour fermer un cathéter péritonéal.
